# EUROPEAN PATENT SPECIFICATION

(11) **EP 4 190 237 B1**
(45) Date of publication and mention of the grant of the patent: **28.10.2026**
(21) Application number: 21854556.4
(22) Date of filing: 31.05.2021
(51) Int. Cl.: A61B 5/145, A61B 5/00

(54) **BODY FLUID ANALYTE DETECTION DEVICE**
VORRICHTUNG ZUM NACHWEIS VON ANALYTEN IN KÖRPERFLÜSSIGKEITEN
DISPOSITIF DE DÉTECTION D'ANALYTES DE LIQUIDES ORGANIQUES

(30) Priority: 03.08.2020 WO PCT/CN2020/106518; 03.08.2020 WO PCT/CN2020/106522
(43) Date of publication of application: 07.06.2023
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/097188
(87) International publication number: WO 2022/028070

(56) References cited:
- EP-B1- 2 332 466
- CN-A- 104 887 242
- CN-A- 106 470 593
- CN-A- 106 470 593
- CN-A- 109 730 674
- CN-A- 109 998 560
- CN-A- 110 584 676
- CN-A- 112 394 097
- CN-U- 202 266 572
- CN-U- 210 204 726
- US-A1- 2018 296 146

## Description

### TECHNICAL FIELD

The invention mainly relates to the field of medical devices, in particular to a body fluid analyte detection device.

### BACKGROUND

The pancreas in a normal human body can automatically monitor the blood glucose level and automatically secrete required amount of insulin/glucagon. In the body of a type 1 diabetes patient, the pancreas does not function properly and cannot produce enough insulin for the body. Therefore, type 1 diabetes is a metabolic disease caused by abnormal pancreatic function, and diabetes is a lifelong disease. At present, there is no cure for diabetes with medical technology. The onset and development of diabetes and its complications can only be controlled by stabilizing blood glucose.

Diabetics need to have their blood glucose measured before they inject insulin into the body. At present, most of the testing methods can continuously measure blood glucose level and transmit the data to a remote device in real time for the user to view. This method is called Continuous Glucose Monitoring (CGM).

The existing body fluid analyte detection devices cannot achieve good waterproof. When users perform underwater activities such as bathing, washing and swimming, water droplets will enter the detection devices. Once they contact with the electrical connection area, short circuit fault or current intensity disturbance will occur, affecting the detection accuracy and reliability. Therefore, users often need to remove the detection devices from their bodies before underwater activities, causing great inconvenience.

CN 109 998 560 A describes a separate power supply blood glucose monitoring transmitter system consisting of a transmitter and a sensor base unit. For assembling the transmitter on the sensor base unit, the transmitter is provided with an edge boss and housing slots. Correspondingly, the sensor base unit is provided with a snap opening and elastic buckles. When the transmitter is installed, the elastic buckles on both sides are outwardly opened, and retract when in place. For deinstalling the transmitter from the sensor base unit, a separate force has to be applied to each elastic buckle engaged with its corresponding snap opening, resulting in a requirement of applying at least two forces in two different directions for separating the transmitter from the sensor base unit. For deinstalling the transmitter from the sensor base unit, separate respective forces have to be applied on the elastic buckles integrated in the sensor base unit.

CN 104 887 242 A describes an analyte sensing system with a transmitter, a bottom shell and a sensor. Two O-Rings provide a waterproof seal between the sensor and the transmitter.

US 2018/296146A1 describes a body fluid analyte detection device with a transmitter, a bottom shell, and a sensor, wherein a sealing ring is provided between the sensor and the transmitter.

Therefore, the prior art urgently needs a body fluid analyte detection device with good waterproof performance.

### BRIEF SUMMARY OF THE INVENTION

The objective of the present invention is solved by a body fluid analytic detection device according to claim 1 and by a continuous glucose monitoring device according to claim 15. Advantageous embodiments are defined by the features of the dependent claims.

The embodiment of the invention discloses a body fluid analyte detection device. The electrical connection area in the body fluid analyte detection device is provided with a waterproof structure, which comprises a groove and a sealing ring arranged on the bottom shell. One end of the sealing ring is placed in the groove, and the other end is in contact with the shell of transmitter, so as to prevent water droplets from entering the electrical connection area, avoiding short circuit and current intensity disturbance. Users do not need to remove the detector from their bodies when bathing, washing, swimming and other underwater actions, thus enhancing the user experience, improve the reliability of detection data.

The invention discloses a body fluid analyte detection device, which comprises: a transmitter which is provided with at least one first clamp part. The bottom shell is provided with a second clamp part corresponding to the first clamp part. Through the first clamp part and the second clamp part, the transmitter can be assembled on the bottom shell. The bottom shell comprises a fixed part and a forced part. When separating the bottom shell and the transmitter, the fixed part is fixed, and the force is applied to the forced part in one direction, the bottom shell fails, and at least one pair of the first and second clamp parts that are clamped together are separated from each other, and further separating the bottom shell from the transmitter. The sensor installed on the bottom shell comprises a sensor and a conductive tape. The sensor is used to detect the parameter information of the body fluid analyte, and is electrically connected with the transmitter through the conductive tape to transmit the parameter signal. The battery is electrically connected with the transmitter to provide electrical energy for the transmitter. The waterproof structure, which comprises a groove and a sealing ring arranged on the bottom shell. The lower end of the sealing ring is placed in the groove, and the upper end contacts the shell of the transmitter to provide waterproof protection for the electrical connection area of the body fluid analyte detection device.

According to one aspect of the invention, the battery is sealed in the transmitter, and the waterproof structure is located in the electrical connection area between the sensor and the transmitter.

According to one aspect of the invention, the battery is sealed in the bottom shell to form a battery sealing chamber, and at least two electrodes are led out from the battery sealing chamber, and the electrodes comprise at least one anode and one cathode.

According to one aspect of the invention, the transmitter is electrically connected with the positive pole and the negative pole respectively to obtain the electric energy of the battery.

According to one aspect of the invention, the waterproof structure is located in the electrical connection area between the transmitter and the anode and cathode.

According to one aspect of the invention, the sealing ring can provide elastic force to promote the separation of the transmitter and the bottom shell.

According to one aspect of the invention, the electrode is an elastic conductive material.

According to one aspect of the invention, the sealing ring is an insulating rubber ring.

According to one aspect of the invention, the diameter of the sealing ring is larger than the inner diameter of the groove.

According to one aspect of the invention, the upper end of the sealing ring is higher than the upper end of the groove.

According to one aspect of the invention, there are two sealing rings, which are respectively located on the upper and lower sides of the sensor.

According to one aspect of the invention, the part of the bottom shell that is equipped with the transmitter is a forced part.

According to one aspect of the invention, the side of the bottom shell is provided with an outward convex part, and the convex part is the forced part.

According to one aspect of the invention, the battery is arranged in the bottom shell, and at least one connection hole is arranged in the bottom shell. Through the connection hole, the transmitter is electrically connected with the two poles of the battery, and the battery part is the forced part.

According to one aspect of the invention, a sealing ring is arranged around the connection hole to seal the electrical connection position. When the force is applied to the forced part, the sealing ring provides the elastic force to promote the separation of the bottom shell and the transmitter.

The invention also discloses a continuous glucose monitoring device, comprising the body fluid analyte detection device described above, and a receiver for receiving the parameter signal transmitted by the transmitter.

Compared with the prior art, the technical scheme of the invention has the following advantages:
In the body fluid analyte detection device disclosed by the invention, waterproof structures are arranged in each electrical connection area to prevent water droplets from entering the electrical connection area, so as to avoid short circuit and current intensity disturbance. On the one hand, users do not need to take the detector off when they are bathing, washing, swimming and other underwater actions, which enhances the user experience, and on the other hand, reduce the interference of detection data jumping caused by current intensity disturbance, improve the reliability of detection data.

Further, the transmitter is provided with at least one first clamp part, the bottom shell is provided with a second clamp part corresponding to the first clamp part, the bottom shell comprises a fixed part and a forced part, when separating the bottom shell and the transmitter, the fixed part is fixed, and the force is applied to the forced part in one direction, the bottom shell fails, and at least one pair of first clamp parts and second clamp parts that are clamped to each other are separated from each other, thereby separating the bottom shell and the transmitter. And a battery for supplying power to the transmitter. Applying force to the forced part of the bottom shell in only one direction can make the bottom shell invalid, thus separating the first engagement part from the second engagement part, and the transmitter can be reused. It also reduces the user's operation steps when separating the transmitter from the bottom shell, and enhances the user experience.

Further, the battery can be directly sealed in the transmitter to provide electrical energy for the transmitter. The waterproof effect between the battery and the transmitter can be achieved without additional waterproof structure, avoiding damage to the transmitter due to short circuit, and improving the reliability of the body fluid analyte detection device.

Further, the battery can be sealed in the bottom shell and discarded with the bottom shell. The user replaces the battery at the same time every time the bottom shell is replaced, and the battery always maintains a high-performance working state. Waterproof structure shall be set in the electrical connection area of transmitter and battery lead out electrode to prevent water droplets from entering the electrical connection area, so as to avoid short circuit and current intensity disturbance.

Further, the electrode is made of conductive elastic materials. When the transmitter contacts with the electrode, the electrode is squeezed to keep the electrode in a continuous compression state. The electrode is retracted into the groove and remains elastic. On the one hand, the electrode keeps close contact with the transmitter to ensure the stability of electric energy transmission. on the other hand, it facilitates the close contact between the shell of transmitter and the sealing ring to improve the waterproof performance, prevent water droplets from entering the electrical connection area, and avoid causing short circuit and current intensity disturbance.

Further, the sealing ring is made of insulating rubber. Since the rubber is flexible and has certain compression elasticity, the transmitter has a certain extrusion force on the sealing ring when it is installed on the chassis, which can better maintain the close contact between the sealing ring and the shell of transmitter, prevent water droplets from entering the electrical connection area, and avoid short circuit and current intensity disturbance.

Further, when the bottom shell fails, the first clamp part and the second clamp part are separated from each other, and the sealing ring can promote the separation of the transmitter and the bottom shell.

Further, the diameter of the sealing ring is larger than the inner diameter of the groove, and the sealing ring can be more tightly fixed in the groove, increasing the reliability of the waterproof structure.

Further, the upper end of the sealing ring is higher than the upper end of the groove, and the shell of transmitter and the sealing ring can be more closely contacted, increasing the reliability of the waterproof structure.

### BRIEF DESCRIPTION OF THE DRAWINGS

Fig. 1a is the structural diagram of a body fluid analyte detection device according to an embodiment of the invention.
Fig. 1b is the structural diagram of a body fluid analyte detection device according to another embodiment of the invention.
Fig. 2 is the three-dimensional structure diagram of a sensor according to an embodiment of the invention.
Fig. 3a is the sectional structure diagram of the sensor electrical connection area according to an embodiment of the invention.
Fig. 3b is the sectional structure diagram of the waterproof structure of the sensor electrical connection area according to an embodiment of the invention.
Fig. 3c is the sectional structure diagram of the waterproof structure of the sensor electrical connection area after the transmitter is installed according to an embodiment of the invention.
Fig. 4 is the top view of a bottom shell with a battery according to an embodiment of the invention.
Fig. 5a shows the sectional structure of the electrode electrical connection area according to an embodiment of the invention.
Fig. 5b is the sectional structure diagram of the waterproof structure of the electrode electrical connection area according to an embodiment of the invention.
Fig. 5c is the sectional structure diagram of the waterproof structure of the electrode electrical connection area after the transmitter is installed according to an embodiment of the invention.
Fig. 6 is the schematic diagram of a continuous glucose monitoring device according to an embodiment of the invention.

### DETAILED DESCRIPTION

As mentioned above, the detection device of the prior art cannot achieve a good waterproof effect. When users perform underwater activities such as bathing, washing and swimming, water droplets will enter the detection device. Once they contact with the electrical connection area, short circuit fault or current intensity disturbance will occur, affecting the accuracy and reliability of detection. Therefore, users often need to remove the detection device from their bodies before underwater activities, causing great inconvenience.

In order to solve this problem, the invention provides a body fluid analyte detection device, which is equipped with a waterproof structure in each electrical connection area to prevent water droplets from entering the electrical connection area, so as to avoid short circuit and current intensity disturbance. When users perform underwater actions such as bathing, washing and swimming, they do not need to take the detector off their bodies, which enhances the user experience and improves the reliability of detection data.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

### First embodiment

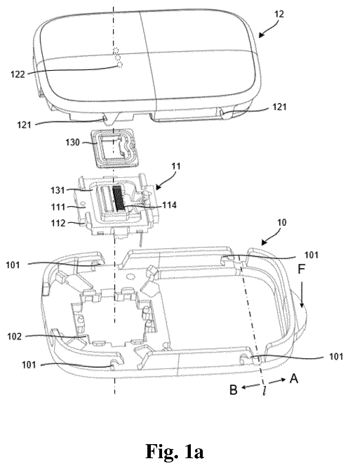

Fig. 1a is the schematic diagram of the three-dimensional structure of the body fluid analyte detection device in the embodiment of the invention.

The detection device comprises the bottom shell 10, the sensor 11 and the transmitter 12.

The bottom shell 10 is used for assembling the transmitter 12 and the sensor 11, and sticking the detection device on the skin surface through the bottom adhesive tape (not shown in the figure). The bottom shell 10 comprises a fixed part and a forced part. The bottom shell 10 is provided with at least one second clamp part 101. The second clamp part 101 is used for engaging the transmitter 12. Specifically, in the embodiment of the invention, the number of the second clamp parts 101 is two, and the two second clamp parts 101 correspond to the side walls of the bottom shell 10.

Here, the fixed part and the forced part are relative concepts. According to the structural design of the bottom shell 10 and the transmitter 12, the positions of the fixed part and the forced part can be selected differently, which will be described in detail below.

In the embodiment of the invention, the connecting line 1 of the two second clamp parts 101 divides the bottom shell 10 into Side A and Side B. Side A is equipped with a forced part, and Side B is equipped with a fixed part.

Therefore, in the embodiment of the invention, the process of separating the bottom shell 10 and the transmitter 12 is as follows: fix the fixed part on the Side B with fingers, and apply a force F to the forced part with another finger in one direction to make the second clamp part 101 invalid, and then separate the second clamp part 101 from the first clamp part 121, so that the transmitter 12 is separated from the bottom shell 10.

In other embodiments of the invention, the number of the second clamp parts 101 is four, and four second clamp parts 101 are correspondingly arranged on the opposite side walls of the bottom shell 10, two on each side.

In other embodiments of the invention, the number of the second clamp parts 101 is six, and the six second clamp parts 101 correspond to the side walls of the bottom shell 10, with two on each side.

The embodiment of the invention does not specifically limit the shape of the top view of the detection device, and its shape can also be rounded rectangle, rectangle, circle, ellipse or other shapes.

The transmitter 12 is provided with at least one first clamp part 121. The first clamp part 121 corresponds to the second clamp part 101. The transmitter 12 is assembled on the bottom shell 10 by clamping the second clamping portion 101 and the first clamping portion 121 to each other. Obviously, in the embodiment of the invention, the transmitter 12 is provided with two first snap portions 121, that is, two pairs of first snap portions 121 and second snap portions 101 that snap together with each other.

Here, the first clamp part 121 corresponds to the second clamp part 101, which means that they are equal in number and corresponding in position.

When separating the bottom shell 10 from the transmitter 12, the fixed part is fixed by a finger or other devices. When another finger or other auxiliary devices are used to apply force to the forced part in one direction, the bottom shell 10 will become invalid. The second clamp part 101 and the first clamp part 121 are separated from each other, thus separating the transmitter 12 from the bottom shell 10. That is, when separating the bottom shell 10 and the transmitter 12, the user can separate them by applying force to the forced part with only one finger in one direction, which is convenient for the user to operate. After separation, the transmitter can be reused, reducing the cost of users.

It should be noted here that failure is a conventional concept in the field of engineering materials. After the failure, the material will lose its original function, and the failed part cannot be restored again. Since the second clamp part 101 is a part of the bottom shell 10, the failure of the bottom shell 10 comprises the failure of the bottom plate, side wall or the second clamp part 101 of the bottom shell 10. Therefore, the failure mode of the bottom shell 10 comprises one or more of the following: the bottom plate or side wall of the bottom shell 10 breaks, the bottom shell 10 breaks, the second clamp part 101 breaks, and the plastic deformation of the bottom shell 10. Obviously, after the bottom shell 10 fails, the bottom shell 10 will lose the function and function of engaging the transmitter 12.

The methods for fixing the fixed part comprise clamping, supporting, etc. There are no specific restrictions here, as long as the conditions for fixing the fixed part can be met.

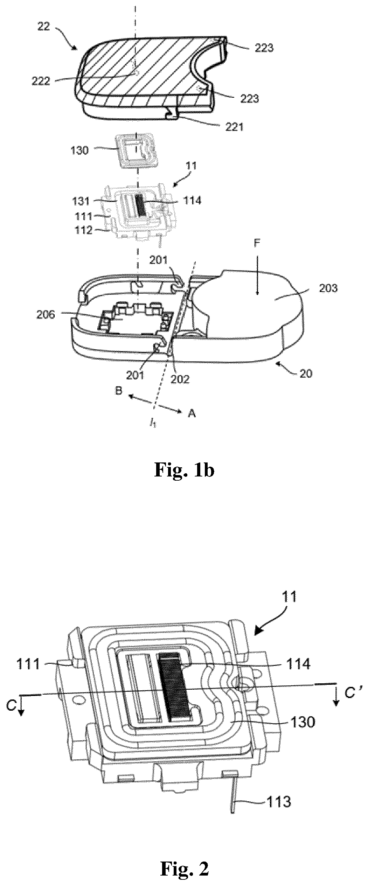

In combination with the three-dimensional structure diagram of the sensor shown in Fig. 2, the sensor 11 is installed on the bottom shell 10, at least comprising the probe 113 and the conductive tape 114. The probe 113 is used to penetrate the human skin, detect the parameter information of the body fluid analyte, and convert it into an electrical signal. The electrical signal is transmitted to the conductive electrode 122 of the transmitter 12 through the conductive tape 114, and the transmitter 12 transmits the parameter information of the body fluid analyte to the user.

In the embodiment of the invention, the battery is located in the shell of the transmitter 12, forming a good waterproof seal. The conductive electrode 122 is electrically connected with the conductive adhesive strip 114. A groove 131 is arranged on the sensor bottom shell 111 and around the conductive adhesive strip 114 to place the sealing ring 130, and the sealing ring contour is consistent with the groove contour.

In other embodiments of the invention, the contour of the sealing ring and the contour of the groove can also be inconsistent, for example, the groove is square, circular, arc or a combination thereof, and the corresponding sealing ring is circular, arc, square or a combination thereof.

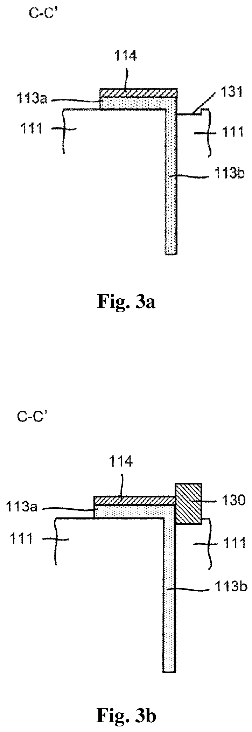

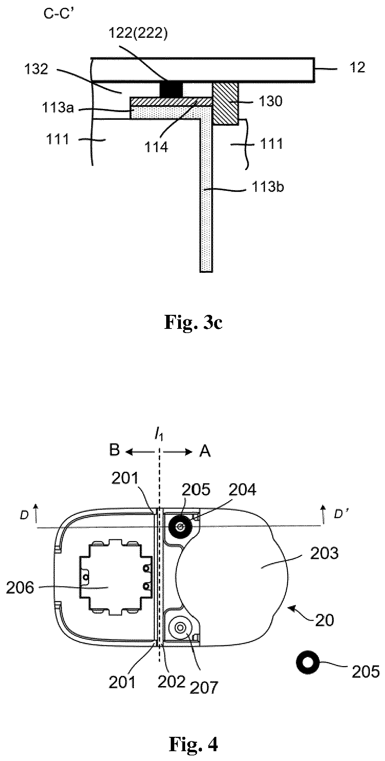

To better understand the waterproof principle of the waterproof structure composed of the groove 131 and the sealing ring 130, refer to Figs 3a, 3b and 3c.

Fig. 3a shows the C-C' profile of the sensor 11 shown in Fig. 2 before installing the sealing ring 130. The groove 131 is arranged on the sensor bottom housing 111, surrounding the probe 113 and the conductive adhesive strip 114. The probe 113 is divided into the internal part 113b and the external part 113a. The external part 113a bends or bends towards the upper end of the bottom shell 111 and is laid flat on the bottom shell 111. Fig. 3b shows the C-C' profile of sensor 11 shown in Fig. 2 after installing sealing ring 131. The contour of sealing ring 130 is consistent with that of groove 131. The sealing ring 130 closely fits groove 131, sensor 13 and conductive adhesive strip 114, and the upper end of sealing ring 130 is slightly higher than the upper end of conductive adhesive strip 114. Here, "slightly higher" means that the upper end of the sealing ring 130 is 0~5mm higher than the upper end of the conductive tape 114, preferably 1mm. Fig. 3c shows the C-C' profile of the sensor 11 shown in Fig. 2 after installing the sealing ring 131 and the transmitter 12. The conductive electrode 122 contacts the conductive tape 114, and the shell of transmitter contacts the upper surface of the sealing ring 130. It can be predicted that the shell of transmitter 12, the sealing ring 130 and the groove 131 can form a sealed chamber 132, and the external part of the probe 113a, the conductive tape 114 and the conductive electrode 122 are located in the chamber 132. When the body fluid analyte detection device enters the underwater, the water drops are blocked by the shell of transmitter 12, the sealing ring 130 and the groove 131, and cannot enter the chamber 132, thus forming a waterproof protection for the electrical connection area of the conductive electrode 122 and the conductive adhesive strip 114.

In other embodiments of the invention, the size of the sealing ring is slightly larger than the size of the groove, so that the sealing ring 130 can be more tightly installed in the groove 131 without easy falling off, and the edge of the sealing ring 130 can form a more closed contact with the groove 131 to achieve better waterproof protection.

In other embodiments of the invention, a layer of sealing ring (not shown in the figure) can also be added below the external part 113a of the sensor and above the bottom shell 111 to form a waterproof structure together with the sealing ring and groove above the external part 113a of the sensor, which can better prevent water droplets from entering the electrical connection area and have a better waterproof effect.

In other embodiments of the invention, the sealing ring material is preferably insulating rubber. Since the rubber is flexible material and has certain compression elasticity, the transmitter 12 has a certain extrusion force on the sealing ring 130 when it is installed on the transmitter 10, which can better maintain the close contact between the sealing ring 130 and the shell of the transmitter 10, prevent water droplets from entering the electrical connection area, and avoid causing short circuit and current intensity disturbance. In the embodiment of the invention, when the bottom shell 10 will fail, the second clamp part 101 and the first clamp part 121 are separated from each other, and the compressive elasticity of the sealing ring 130 can promote the separation of the bottom shell 10 and the transmitter 12.

### Second embodiment

Fig. 1b is the structure diagram of another embodiment of the invention.

The detection device comprises the bottom shell 20, the sensor 11 and the transmitter 22.

In the embodiment of the invention, the fixed part and the forced part are relative concepts. According to the structural design of the bottom shell 20 and the transmitter 22, the positions of the fixed part and the forced part can be selected differently.

In the embodiment of the invention, the connecting line 11 of the two second clamp parts 202 divides the bottom shell 20 into Side A and Side B. Side A is equipped with a forced part, and Side B is equipped with a fixed part.

Therefore, in the embodiment of the invention, the process of separating the bottom shell 20 and the transmitter 22 is as follows: fix the fixed part at side B with a finger, and apply force F to the forced part with another finger in one direction to make the second clamp part 202 invalid, and then separate the second clamp part 202 from the first clamp part 221, so as to separate the transmitter 22 from the bottom shell 20.

In the embodiment of the invention, the number of the second clamp parts 202 is two, and the two second clamp parts 202 correspond to the side walls of the bottom shell 10.

In other embodiments of the invention, the number of the second clamp parts 202 is four, and the four second clamp parts 202 correspond to the opposite side walls of the bottom shell 10, with two on each side.

In other embodiments of the invention, the number of the second clamp parts 202 is six, and the six second clamp parts 202 correspond to the side walls of the bottom shell 10, with two on each side.

The embodiment of the invention does not specifically limit the shape of the top view of the detection device, and its shape can also be rounded rectangle, rectangle, circle, ellipse or other shapes.

The detection device of the embodiment of the invention also comprises a battery 208. The battery 208 is used for supplying power to the transmitter and is arranged in the bottom shell 20. The part where the battery 208 is arranged in the bottom shell 20 is the battery sealing chamber 203. In this way, the battery 208 can be replaced at the same time each time the bottom shell 20 is replaced. The transmitter 22 can be reused because the battery is no longer set, reducing the cost of replacing the transmitter 22. At the same time, the bottom shell 20 always uses a new high-performance battery, which can ensure the continuous high-performance working state of the transmitter 22.

Preferably, in the embodiment of the invention, the top of the battery sealing chamber 203 is flush with the top of the transmitter 22, which can reduce the thickness size of the detection device.

The battery sealing chamber 203 can be directly used as the forced part, so the battery is arranged on the Side A of 11. As the battery sealing chamber 203 is relatively thick and has a relatively large area, as a forced part, it is easier for users to apply the force on the battery sealing chamber 203 to optimize the user's operation steps.

Fig. 4 is the top view of the bottom shell 20.

Since the battery 208 needs to supply power to the transmitter 22, in the embodiment of the invention, the bottom shell 20 is also provided with at least two electrodes 204. The electrical contact 223 of the transmitter 22 is electrically connected with the positive and cathodes of the battery through the electrode 204 to form an electrical connection area. The battery 208 supplies power to the transmitter through the electrode 204 and the electrical contact 223. Once the electrical connection area enters the water drop, it causes a short circuit, which causes the unstable power supply of the battery 208. The current intensity received by the transmitter 22 fluctuates, which may cause the transmitter 22 to receive the body fluid analyte parameter information of the probe 113 and the transmitted parameter information to jump, affecting the reliability of the analyte detection device. Therefore, waterproof protection is required for the electrical connection area. The waterproof structure of the electrical connection area comprises a groove 207 and a sealing ring 205.

In other embodiments of the invention, the contour of the sealing ring and the contour of the groove can also be inconsistent, for example, the groove is square, circular, arc or a combination thereof, and the corresponding sealing ring is circular, arc, square or a combination thereof.

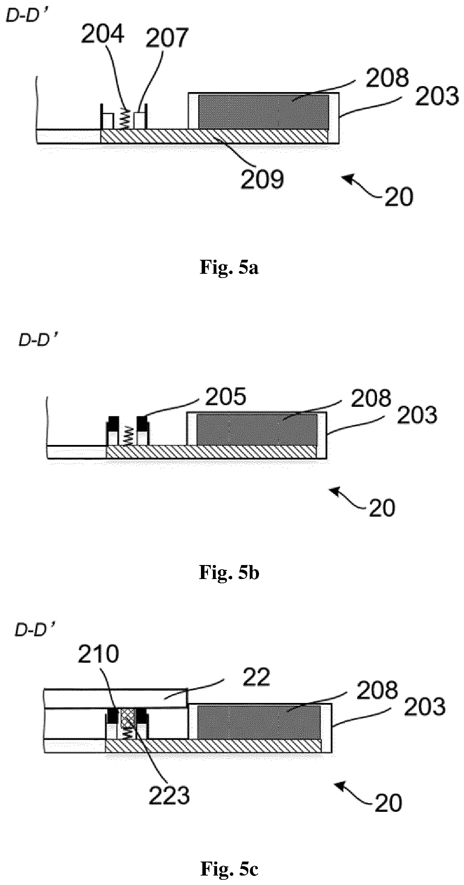

To better understand the waterproof principle of waterproof structure composed of groove 207 and sealing ring 205, refer to Fig. 5a, Fig. 5b and Fig. 5c.

Fig. 5a shows the D-D' profile of the bottom shell 20 shown in Fig. 4 before the installation of the sealing ring 205. The battery 208 is placed in the battery sealing chamber 203. The battery sealing chamber 203 is composed of the anode and cathode conductive strips 209 of the battery 208 and the shell of the bottom shell 20, forming a completely enclosed space. Water droplets cannot enter the battery sealing chamber. The anode and cathode conductive strips 209 continue to extend outward to the battery sealing chamber 203 to the groove 207 and cover the bottom end of the groove 207, the electrode 204 is located in the middle of the groove 207, and one end is fixed on the positive and cathode conductive strip 209. Fig. 5b shows the D-D' profile of the bottom shell 20 shown in Fig. 4 after the installation of the sealing ring 205. The sealing ring 205 is located on the upper end of the groove 207, its contour is consistent with the groove contour, and can envelope the electrode 204. The upper end of the sealing ring 205 is slightly higher than the upper end of the groove 207. Here, "slightly higher" means that the upper end of the sealing ring 205 is 0~5mm higher than the upper end of the groove 207, preferably 1mm.

Fig. 5c shows the D-D' profile of the bottom shell 20 shown in Fig. 4 after the installation of the sealing ring 205 and the transmitter 22. The transmitter power electrode 223 contacts the electrode 204 to obtain the electric energy of the battery 208. The shell of the transmitter 22 contacts the upper surface of the sealing ring 205. It can be predicted that the shell of transmitter 22, the sealing ring 205, the groove 207 and the anode and cathode conductive strip 209 form a sealing chamber 210, and the transmitter power electrode 223 and the electrode 204 are located in the sealing chamber 210. When the body fluid analyte detection device enters the underwater, the water drops are blocked by the transmitter 22 shell, the sealing ring 205 and the groove 207, and cannot enter the chamber 210, thus forming a waterproof protection for the electrical connection area of the transmitter power electrode 223, the electrode 204, and the anode and cathode conductive strips 209.

In other embodiments of the invention, the size of the sealing ring is slightly larger than the size of the groove, so that the sealing ring 205 can be more tightly installed in the groove 207, and is not easy to fall off, and the edge of the sealing ring 205 can form a more closed contact with the groove 207, so as to achieve better waterproof protection.

In other embodiments of the invention, the electrode 204 is an elastic conductive material that can be electrically connected with the transmitter power electrode 223, for example, it can be a conductive spring or a conductive spring. When the transmitter 22 is installed on the bottom shell 20, the transmitter power electrode 223 squeezes the electrode 204, making the electrode 204 continuously compressed and elastic, so that the electrode 204 can maintain continuous close contact with the transmitter power electrode 223, Ensure that the battery 208 delivers stable electrical energy to the transmitter 22.

In other embodiments of the invention, the sealing ring is preferably made of insulating rubber. Since the rubber is flexible and has certain compression elasticity, the transmitter 22 has a certain extrusion force on the sealing ring 205 when it is installed on the chassis 20, which can better maintain the close contact between the sealing ring 205 and the shell 20 of transmitter, prevent water droplets from entering the electrical connection area, and avoid causing short circuit and current intensity disturbance.

In the embodiment of the invention, the waterproof structure of the conductive electrode 222 of the sensor 11 and the transmitter 22 is the same as that of the first embodiment, so it will not be repeated here.

In the embodiment of the invention, the extruded sealing ring 105 exerts a certain elastic force on the transmitter 22. When the forced part is applied with a force F, the sealing ring 105 provides the elastic force to promote the separation of the transmitter 22 from the bottom shell 20.

Preferably, in the embodiment of the invention, in order to seal the connection position between the transmitter 22 and the sensor, a sealing ring is arranged around the sensor. Similar to the above sealing ring 105, when the bottom shell 20 will fail, the second clamp part 201 and the first clamp part 221 will be separated from each other, and the compressive elasticity of the sealing rings 130 and 205 can promote the separation of the bottom shell 10 and the transmitter 12.

To sum up, the invention discloses a body fluid analyte detection device, in which a waterproof structure is arranged in the electrical connection area of the device to prevent water droplets from entering the electrical connection area, so as to avoid short circuit and current intensity disturbance. When users perform underwater actions such as bathing, washing, swimming, etc., they do not need to take the detector off, which enhances the user experience and improves the reliability of the detection data.

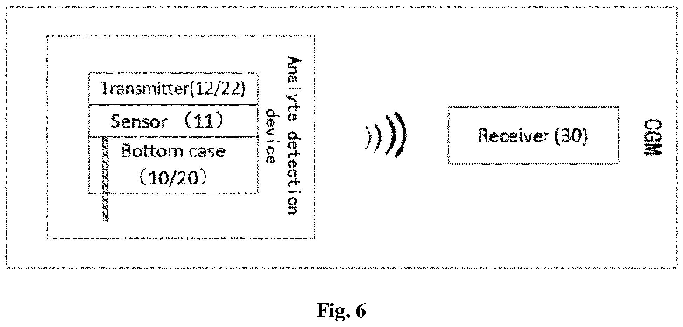

The invention also discloses a continuous glucose monitoring device. As shown in Fig. 6, after the sensor 11 is installed on the transmitter 10 (20), and the transmitter 12 (22) is installed on the chassis 20, the transmitter 12 (22) can obtain the detection data indication signal of the sensor 11, and the transmitter 12 (22) is connected with the wireless data of the receiver 30 to transmit the detection data indication signal to the receiver 30 for the user to know the detection data.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope of the invention. The scope of the invention is limited by the attached claims.

## Claims

1. A body fluid analyte detection device, comprising:
a transmitter (12, 22) which is provided with at least two first clamp parts (121, 221);
a bottom shell (10, 20) which is provided with second clamp parts (101, 201, 202) corresponding to the first clamp parts (121, 221), wherein the transmitter (12, 22) is assembled on the bottom shell (10, 20) by the first clamp parts (121, 221) and the second clamp parts (101, 201, 202) clamped together, the bottom shell (10, 20) comprises a fixed part (B) and a forced part (A), wherein a connecting line (1, 11) of two of the second clamp parts (101, 201, 202) divides the bottom shell (10, 20) into a side A equipped with the forced part (A) and a side B equipped with the fixed part (B), the device is configured such that separating the bottom shell (10, 20) and the transmitter (12, 22) can be done by fixing the fixed part (B) and applying a force (F) to the forced part (A) with only one finger of a user in only one direction, and the device is configured such that by separating the bottom shell (10, 20) from the transmitter (12, 22) the bottom shell (10, 20) is in a failure mode, the failure mode comprising one or more combinations of bottom shell fracture, fracture of the bottom plate or side wall of the bottom shell (10, 20), fracture of the second clamp part (101, 201, 202), and plastic deformation of the bottom shell (10, 20), so that when the bottom shell (10, 20) is in failure mode, the bottom shell (10, 20) cannot be restored again, at least one pair of the first and second clamp parts that are clamped together are separated from each other, and the device is configured such that after separation the transmitter (12, 22) can be reused;
a sensor (11) installed on the bottom shell (10, 20), wherein the sensor (11) comprises a probe (113) and a conductive tape (114), the probe (113) is configured to detect a parameter information of body fluid analyte, and is electrically connected with the transmitter (12, 22) through the conductive tape (114) to transmit a parameter signal;
a battery (208) electrically connected with the transmitter (12, 22) to provide electrical energy for the transmitter (12, 22); and
a waterproof structure, comprising a groove (131, 207) and a sealing ring (130, 205) arranged on the bottom shell (10, 20), wherein a lower end of the sealing ring (130, 205) is placed in the groove (131, 207), and an upper end contacts a shell of the transmitter (12, 22) to provide waterproof protection for electrical connection areas of the body fluid analyte detection device,
wherein the sealing ring (130, 205) provides elastic force to promote the separation of the bottom shell (10, 20) and the transmitter (12, 22).

2. The body fluid analyte detection device of claim 1, wherein the battery is sealed in the transmitter (12).

3. The body fluid analyte detection device of claim 1, wherein the battery (208) is sealed in the bottom shell (20) to form a battery sealing chamber (203), and at least two electrodes (204) are led out from the battery sealing chamber (203), and the electrodes (204) at least comprise one anode and one cathode, wherein, in particular, the transmitter (22) is electrically connected with the anode and the cathode respectively to obtain electric energy of the battery (208), and, in particular, wherein the waterproof structure is located in the electrical connection areas in the transmitter and the anode and cathode.

4. The body fluid analyte detection device of claim 2, wherein the waterproof structure is located in the electrical connection areas in the sensor (11) and the transmitter (12, 22).

5. The body fluid analyte detection device of claim 4, wherein when the force (F) is applied to the forced part (A), the sealing ring (130, 205) provides elastic force to promote separation of the transmitter (12, 22) and the bottom shell (10, 20).

6. The body fluid analyte detection device of claim 3, wherein the electrodes (204) are an elastic conductive material.

7. The body fluid analyte detection device of claim 1, wherein the sealing ring (130, 205) is an insulating rubber ring.

8. The body fluid analyte detection device of claim 1, wherein a diameter of the sealing ring (130, 205) is larger than an inner diameter of the groove (131, 207).

9. The body fluid analyte detection device of claim 1, wherein the upper end of the sealing ring (130. 205) is higher than an upper end of the groove (131, 207).

10. The body fluid analyte detection device of claim 1, wherein there are two sealing rings, which are respectively located on an upper side and a lower side of the probe (113).

11. The body fluid analyte detection device of claim 1, wherein a part of the bottom shell (10. 20) that is equipped with the transmitter (12, 22) is the forced part (A).

12. The body fluid analyte detection device of claim 1, wherein a side of the bottom shell (10, 20) is provided with a convex part which is outward, and the convex part is the forced part (A).

13. The body fluid analyte detection device of claim 1, wherein the battery (208) is arranged in the bottom shell (20), and at least one connection hole is arranged in the bottom shell (20), the transmitter (22) is electrically connected with the two electrodes (204) of the battery through the connection hole, and a battery part (208) is the forced part (A).

14. The body fluid analyte detection device of claim 13, wherein the sealing ring (205) is arranged around the connection hole to seal an electrical connection position, when the force (F) is applied to the forced part (A), the sealing ring (205) provides elastic force to promote separation of the bottom shell (20) and the transmitter (22).

15. A continuous glucose monitoring system, comprising:
a body fluid analyte detection device of claims 1; and
a receiver for receiving the parameter signal transmitted by the transmitter (12, 22).

## Patentansprüche

1. Vorrichtung zum Nachweis von Analyten in Körperflüssigkeiten, umfassend:
einen Sender (12, 22), der mit mindestens zwei ersten Klemmteilen (121, 221) versehen ist;
eine Unterschale (10, 20), die mit zweiten Klemmteilen (101, 201, 202) versehen ist, die den ersten Klemmteilen (121, 221) entsprechen, wobei der Sender (12, 22) durch Zusammenklemmen der ersten Klemmteile (121, 221) und der zweiten Klemmteile (101, 201, 202) an der Unterschale (10, 20) montiert ist, die Unterschale (10, 20) einen fixierten Teil (B) und einen kraftbeaufschlagten Teil (A) umfasst, wobei eine Verbindungslinie (l, l1) von zwei der zweiten Klemmteile (101, 201, 202) die Unterschale (10, 20) in eine Seite A, die mit dem kraftbeaufschlagten Teil (A) versehen ist, und eine Seite B, die mit dem fixierten Teil (B) versehen ist, unterteilt, die Vorrichtung derart eingerichtet ist, dass das Trennen der Unterschale (10, 20) und des Senders (12, 22) durchgeführt werden kann, indem der fixierte Teil (B) fixiert wird und mit nur einem Finger eines Benutzers in nur einer Richtung eine Kraft (F) auf den kraftbeaufschlagten Teil (A) ausgeübt wird, und die Vorrichtung derart eingerichtet ist, dass sich die Unterschale (10, 20) durch das Trennen der Unterschale (10, 20) von dem Sender (12, 22) in einem Ausfallzustand befindet, wobei der Ausfallzustand eine oder mehrere Kombinationen aus einem Bruch der Unterschale, einem Bruch der Bodenplatte oder der Seitenwand der Unterschale (10, 20), einem Bruch des zweiten Klemmteils (101, 201, 202) und einer plastischen Verformung der Unterschale (10, 20) umfasst, sodass, wenn sich die Unterschale (10, 20) in dem Ausfallzustand befindet, die Unterschale (10, 20) nicht wiederhergestellt werden kann, mindestens ein Paar der miteinander verklemmten ersten und zweiten Klemmteile voneinander getrennt ist und die Vorrichtung derart eingerichtet ist, dass der Sender (12, 22) nach dem Trennen wiederverwendet werden kann;
einen an der Unterschale (10, 20) angebrachten Sensor (11), wobei der Sensor (11) eine Sonde (113) und ein leitfähiges Band (114) umfasst, die Sonde (113) dazu eingerichtet ist, Parameterinformationen eines Analyten in einer Körperflüssigkeit zu erfassen, und über das leitfähige Band (114) elektrisch mit dem Sender (12, 22) verbunden ist, um ein Parametersignal zu übertragen;
eine Batterie (208), die elektrisch mit dem Sender (12, 22) verbunden ist, um den Sender (12, 22) mit elektrischer Energie zu versorgen; und
eine wasserdichte Struktur, die eine Nut (131, 207) und einen an der Unterschale (10, 20) angeordneten Dichtungsring (130, 205) umfasst, wobei ein unteres Ende des Dichtungsrings (130, 205) in der Nut (131, 207) angeordnet ist und ein oberes Ende ein Gehäuse des Senders (12, 22) berührt, um elektrische Verbindungsbereiche der Vorrichtung zum Nachweis von Analyten in Körperflüssigkeiten wasserdicht zu schützen,
wobei der Dichtungsring (130, 205) eine elastische Kraft bereitstellt, um das Trennen der Unterschale (10, 20) und des Senders (12, 22) zu fördern.

2. Vorrichtung zum Nachweis von Analyten in Körperflüssigkeiten nach Anspruch 1, wobei die Batterie in dem Sender (12) abgedichtet ist.

3. Vorrichtung zum Nachweis von Analyten in Körperflüssigkeiten nach Anspruch 1, wobei die Batterie (208) in der Unterschale (20) abgedichtet ist, um eine Batteriedichtungskammer (203) zu bilden, und mindestens zwei Elektroden (204) aus der Batteriedichtungskammer (203) herausgeführt sind und die Elektroden (204) mindestens eine Anode und eine Kathode umfassen, wobei insbesondere der Sender (22) jeweils elektrisch mit der Anode und der Kathode verbunden ist, um elektrische Energie der Batterie (208) zu beziehen, und wobei insbesondere die wasserdichte Struktur in den elektrischen Verbindungsbereichen zwischen dem Sender und der Anode und der Kathode angeordnet ist.

4. Vorrichtung zum Nachweis von Analyten in Körperflüssigkeiten nach Anspruch 2, wobei die wasserdichte Struktur in den elektrischen Verbindungsbereichen zwischen dem Sensor (11) und dem Sender (12, 22) angeordnet ist.

5. Vorrichtung zum Nachweis von Analyten in Körperflüssigkeiten nach Anspruch 4, wobei der Dichtungsring (130, 205), wenn die Kraft (F) auf den kraftbeaufschlagten Teil (A) ausgeübt wird, eine elastische Kraft bereitstellt, um das Trennen des Senders (12, 22) und der Unterschale (10, 20) zu fördern.

6. Vorrichtung zum Nachweis von Analyten in Körperflüssigkeiten nach Anspruch 3, wobei die Elektroden (204) aus einem elastischen leitfähigen Material bestehen.

7. Vorrichtung zum Nachweis von Analyten in Körperflüssigkeiten nach Anspruch 1, wobei der Dichtungsring (130, 205) ein isolierender Gummiring ist.

8. Vorrichtung zum Nachweis von Analyten in Körperflüssigkeiten nach Anspruch 1, wobei ein Durchmesser des Dichtungsrings (130, 205) größer als ein Innendurchmesser der Nut (131, 207) ist.

9. Vorrichtung zum Nachweis von Analyten in Körperflüssigkeiten nach Anspruch 1, wobei das obere Ende des Dichtungsrings (130, 205) höher als ein oberes Ende der Nut (131, 207) ist.

10. Vorrichtung zum Nachweis von Analyten in Körperflüssigkeiten nach Anspruch 1, wobei zwei Dichtungsringe vorhanden sind, die jeweils auf einer Oberseite und einer Unterseite der Sonde (113) angeordnet sind.

11. Vorrichtung zum Nachweis von Analyten in Körperflüssigkeiten nach Anspruch 1, wobei ein Teil der Unterschale (10, 20), der mit dem Sender (12, 22) versehen ist, der kraftbeaufschlagte Teil (A) ist.

12. Vorrichtung zum Nachweis von Analyten in Körperflüssigkeiten nach Anspruch 1, wobei eine Seite der Unterschale (10, 20) mit einem nach außen gerichteten konvexen Teil versehen ist und der konvexe Teil der kraftbeaufschlagte Teil (A) ist.

13. Vorrichtung zum Nachweis von Analyten in Körperflüssigkeiten nach Anspruch 1, wobei die Batterie (208) in der Unterschale (20) angeordnet ist und mindestens eine Verbindungsöffnung in der Unterschale (20) angeordnet ist, der Sender (22) durch die Verbindungsöffnung elektrisch mit den zwei Elektroden (204) der Batterie verbunden ist und ein Batterieteil (208) der kraftbeaufschlagte Teil (A) ist.

14. Vorrichtung zum Nachweis von Analyten in Körperflüssigkeiten nach Anspruch 13, wobei der Dichtungsring (205) um die Verbindungsöffnung herum angeordnet ist, um eine elektrische Verbindungsstelle abzudichten, wobei der Dichtungsring (205), wenn die Kraft (F) auf den kraftbeaufschlagten Teil (A) ausgeübt wird, eine elastische Kraft bereitstellt, um das Trennen der Unterschale (20) und des Senders (22) zu fördern.

15. System zur kontinuierlichen Glukoseüberwachung, umfassend:
eine Vorrichtung zum Nachweis von Analyten in Körperflüssigkeiten nach Anspruch 1; und
einen Empfänger zum Empfangen des von dem Sender (12, 22) übertragenen Parametersignals.

## Revendications

1. Dispositif de détection d'analytes de liquides organiques, comprenant :
un émetteur (12, 22) qui est pourvu d'au moins deux premières parties de serrage (121, 221) ;
une coque inférieure (10, 20) qui est pourvue de deuxièmes parties de serrage (101, 201, 202) correspondant aux premières parties de serrage (121, 221), dans lequel l'émetteur (12, 22) est assemblé sur la coque inférieure (10, 20) par serrage conjoint des premières parties de serrage (121, 221) et des deuxièmes parties de serrage (101, 201, 202), la coque inférieure (10, 20) comprend une partie fixe (B) et une partie sollicitée (A), dans lequel une ligne de liaison (l, l1) de deux des deuxièmes parties de serrage (101, 201, 202) divise la coque inférieure (10, 20) en un côté A pourvu de la partie sollicitée (A) et un côté B pourvu de la partie fixe (B), le dispositif est configuré de telle sorte que la séparation de la coque inférieure (10, 20) et de l'émetteur (12, 22) puisse être réalisée en maintenant la partie fixe (B) et en appliquant une force (F) à la partie sollicitée (A) avec un seul doigt d'un utilisateur dans une seule direction, et le dispositif est configuré de telle sorte que, lors de la séparation de la coque inférieure (10, 20) de l'émetteur (12, 22), la coque inférieure (10, 20) se trouve dans un état de défaillance, l'état de défaillance comprenant une ou plusieurs combinaisons parmi une rupture de la coque inférieure, une rupture de la plaque inférieure ou de la paroi latérale de la coque inférieure (10, 20), une rupture de la deuxième partie de serrage (101, 201, 202) et une déformation plastique de la coque inférieure (10, 20), de sorte que, lorsque la coque inférieure (10, 20) se trouve dans l'état de défaillance, la coque inférieure (10, 20) ne peut pas être remise en état, au moins une paire constituée par les première et deuxième parties de serrage serrées ensemble est séparée l'une de l'autre, et le dispositif est configuré de telle sorte que, après la séparation, l'émetteur (12, 22) puisse être réutilisé ;
un capteur (11) installé sur la coque inférieure (10, 20), dans lequel le capteur (11) comprend une sonde (113) et une bande conductrice (114), la sonde (113) est configurée pour détecter des informations relatives à un paramètre d'un analyte de liquide organique et est reliée électriquement à l'émetteur (12, 22) par l'intermédiaire de la bande conductrice (114) afin de transmettre un signal de paramètre ;
une batterie (208) reliée électriquement à l'émetteur (12, 22) afin de fournir de l'énergie électrique à l'émetteur (12, 22) ; et
une structure étanche comprenant une rainure (131, 207) et une bague d'étanchéité (130, 205) disposées sur la coque inférieure (10, 20), dans lequel une extrémité inférieure de la bague d'étanchéité (130, 205) est placée dans la rainure (131, 207), et une extrémité supérieure est en contact avec une coque de l'émetteur (12, 22) afin d'assurer une protection étanche des zones de connexion électrique du dispositif de détection d'analytes de liquides organiques,
dans lequel la bague d'étanchéité (130, 205) fournit une force élastique favorisant la séparation de la coque inférieure (10, 20) et de l'émetteur (12, 22).

2. Dispositif de détection d'analytes de liquides organiques selon la revendication 1, dans lequel la batterie est scellée dans l'émetteur (12).

3. Dispositif de détection d'analytes de liquides organiques selon la revendication 1, dans lequel la batterie (208) est scellée dans la coque inférieure (20) afin de former une chambre étanche de batterie (203), et au moins deux électrodes (204) sont amenées hors de la chambre étanche de batterie (203), et les électrodes (204) comprennent au moins une anode et une cathode, dans lequel, en particulier, l'émetteur (22) est relié électriquement respectivement à l'anode et à la cathode afin de recevoir l'énergie électrique de la batterie (208), et, en particulier, dans lequel la structure étanche est située dans les zones de connexion électrique entre l'émetteur et l'anode et la cathode.

4. Dispositif de détection d'analytes de liquides organiques selon la revendication 2, dans lequel la structure étanche est située dans les zones de connexion électrique entre le capteur (11) et l'émetteur (12, 22).

5. Dispositif de détection d'analytes de liquides organiques selon la revendication 4, dans lequel, lorsque la force (F) est appliquée à la partie sollicitée (A), la bague d'étanchéité (130, 205) fournit une force élastique favorisant la séparation de l'émetteur (12, 22) et de la coque inférieure (10, 20).

6. Dispositif de détection d'analytes de liquides organiques selon la revendication 3, dans lequel les électrodes (204) sont constituées d'un matériau conducteur élastique.

7. Dispositif de détection d'analytes de liquides organiques selon la revendication 1, dans lequel la bague d'étanchéité (130, 205) est une bague en caoutchouc isolant.

8. Dispositif de détection d'analytes de liquides organiques selon la revendication 1, dans lequel un diamètre de la bague d'étanchéité (130, 205) est supérieur à un diamètre intérieur de la rainure (131, 207).

9. Dispositif de détection d'analytes de liquides organiques selon la revendication 1, dans lequel l'extrémité supérieure de la bague d'étanchéité (130, 205) est plus haute qu'une extrémité supérieure de la rainure (131, 207).

10. Dispositif de détection d'analytes de liquides organiques selon la revendication 1, dans lequel deux bagues d'étanchéité sont respectivement situées sur un côté supérieur et un côté inférieur de la sonde (113).

11. Dispositif de détection d'analytes de liquides organiques selon la revendication 1, dans lequel une partie de la coque inférieure (10, 20) qui est pourvue de l'émetteur (12, 22) est la partie sollicitée (A).

12. Dispositif de détection d'analytes de liquides organiques selon la revendication 1, dans lequel un côté de la coque inférieure (10, 20) est pourvu d'une partie convexe dirigée vers l'extérieur, et la partie convexe est la partie sollicitée (A).

13. Dispositif de détection d'analytes de liquides organiques selon la revendication 1, dans lequel la batterie (208) est disposée dans la coque inférieure (20), et au moins un trou de connexion est disposé dans la coque inférieure (20), l'émetteur (22) est relié électriquement aux deux électrodes (204) de la batterie à travers le trou de connexion, et une partie de batterie (208) est la partie sollicitée (A).

14. Dispositif de détection d'analytes de liquides organiques selon la revendication 13, dans lequel la bague d'étanchéité (205) est disposée autour du trou de connexion afin d'assurer l'étanchéité d'une position de connexion électrique, lorsque la force (F) est appliquée à la partie sollicitée (A), la bague d'étanchéité (205) fournit une force élastique favorisant la séparation de la coque inférieure (20) et de l'émetteur (22).

15. Système de surveillance continue du glucose, comprenant :
un dispositif de détection d'analytes de liquides organiques selon la revendication 1 ; et
un récepteur destiné à recevoir le signal de paramètre transmis par l'émetteur (12, 22).
